# EUROPEAN PATENT APPLICATION

(11) **EP 0 836 857 A1**
(43) Date of publication of application: **22.04.1998**
(21) Application number: 97402435.8
(22) Date of filing: 15.10.1997
(51) Int. Cl.: A61L 9/12, A61L 9/03, A01M 1/20

(54) **Device for the diffusion of a volatile product with a super-hydrophobic membrane**

(30) Priority: 15.10.1996 FR 9612552
(71) Applicant: MILLIPORE S.A., F-67120 Molsheim (FR)
(72) Inventor: Jallerat, Eric, 92420 Ville D'Avray (FR)
(74) Representative: Rinuy, Santarelli

(57) **Abstract**

A subject of the present invention is a device for the diffusion of a volatile product, such as a perfume, in liquid phase.

This device is characterized by the use of a microporous polymeric membrane (4) with a pore diameter comprised between 0.1 and 5 micrometers and rendered super-hydrophobic so as not to be wetted by the liquid phase.

This membrane can constitute a vertical wall of a container (3) containing the liquid phase.

## Description

The present invention relates to a device for the diffusion of a volatile product such as a perfume, a deodorant, an insecticide, a disinfectant product or a bactericidal product.

Such a device in which the volatile product is present in liquid form or dissolved in an appropriate solvent poses practical difficulties.

In fact, the liquid form of the volatile product, which is the preferred form for manufacturers as it allows extractions, blending, dosing and transfers, is not so for users as it necessitates frequent spraying and presents a risk of leakage with, in the case of a perfume to be sprayed on oneself, additional risks of interaction with the skin or staining of clothing.

For this reason, in use inside a house or a car, the volatile product to be diffused is preferably presented in a solid form, in order to avoid any risk of being spilt, but such a form has the major drawback of running out rapidly.

To avoid the problems connected with the use of a volatile product in solid form, it has been attempted to promote devices for the diffusion of a volatile product presented in liquid form in a container.

When the container is a bottle to be opened, the diffusion of the volatile product is limited by the neck of the bottle, which generally is of a cross section which is too small to allow sufficient evaporation of this product and the risks of tipping the container over are not insignificant.

Attempts were made to overcome these drawbacks by proposing containers having a porous membrane through which the volatile product can diffuse.

However, these realizations did not meet with the anticipated success because the porous membrane, whilst hydrophobic, is moistened by the liquid phase containing the volatile product, resulting in a risk of leakage, or presents an extremely fine porous structure, of the order of a pore diameter comprised between 0.001 and 0.01 micrometer, of the same order of magnitude as the molecules of the volatile product, actively limiting its diffusion by checking, hindering or selective retardation, which necessitates a membrane support and renders the membrane particularly costly.

The problem to be resolved is particularly difficult in the case of diffusers for perfumes.

During the manufacture of perfumes, either of rare fragrances for persons or of more industrial perfumes for homes or cars, most of the manufacturing phases are carried out using the liquid form. For luxury perfumes, extractions - often from flowers or plants - are carried out in liquid phase. This also applies to industrial perfumes, odoriferous molecules are synthesised or extracted in liquid-liquid interactions.

Most odoriferous molecules are normally very volatile, i.e., with a high vapour pressure. There is always an equilibrium between the liquid phase and the gas phase. This equilibrium is generally rapidly displaced towards the gas phase by increasing the temperature. For manufacturers, the liquid form is the easiest to handle in closed tanks in order to minimise losses. Liquid forms based on water, solvent, oil and alcohol are easy to blend, dose and transfer. Manufacturers can thus easily reach the perfume and intensity they wish from a liquid form. While the liquid form is the most commonly used by manufacturers, nevertheless it is not the preferred form for the end user.

In the case of perfumes for people, the difficulty for the manufacturer is to create a liquid which will have a high perfuming intensity, will not change the odour too much on contact with the skin and which will not create any allergic reaction and will not stain clothes. In all cases, the intensity of the perfume is significant at the time of spraying and decreases rapidly as evaporation from the skin advances. During the day, perfume must be applied several times if one wishes to maintain the same odoriferous intensity about one.

In the case of perfumes for the house or car, the problem is the same, the liquid form being the best, but it is often the most difficult to use.

The diffusion of perfumes from the liquid phase, once the volatility of the chosen molecules is known, is controlled by the temperature which will increase the number of molecules passing from the liquid state to the gas state, via the liquid/air interface, which is the surface of the liquid in contact with the ambient air which, by distancing the gas molecules from the surface of the liquid, reduces their concentration at the liquid/gas interface and displaces the equilibrium towards the gas phase of these molecules.

At ambient temperature and with no specific ventilation, it is the surface of the liquid in contact with the atmosphere which will be the determining parameter as regards the concentration of gas molecules and therefore the odoriferous intensity which will be obtained.

A container, such as an open bottle, often presents a liquid interface which is too small and it is very difficult to design containers with a very large liquid surface, due to their bulk and the risk of being tipped over.

The majority of manufacturers use perfumes incorporated in a solid phase (gels, papers...) which are more difficult to manufacture and implement, and which above all are not long-lasting due to their lack of reserves.

An object of the present invention is to overcome these drawbacks by proposing a diffusion device for a volatile product presented in liquid form in a container, in which one of the walls of the container, in contact on one side with the liquid phase where the volatile product is to be found and the other side is in contact with the outside where one wishes to diffuse the volatile product, includes a polymeric microporous membrane, of which the diameter of the pores is comprised between 0.1 and 5 micrometers and which has undergone a treatment to render said membrane super-hydrophobic and oleophobic.

The devices which are the object of the present invention are distinguished from the devices of the prior art, in particular by the following characteristics:

They use polymeric microporous membranes the surface of which has been modified by an appropriate treatment so as not to be wetted in the presence of the usual solvents, such as ethanol or isopropanol, even at temperatures such as those of the human body, which can range up to 37°C and even more.

Such a treatment consists of forming a cross-linked polymer in situ over all the surface of a membrane corresponding to the prescribed pore diameter, obtained for example by saturating the membrane in a reactive system containing a non-saturated ethylenic monomer and having at least one fluoroalkyl group, a cross-linking agent and optionally a polymerization initiator and dissolved in a non-polar or weakly polar solvent system and by subjecting the membrane thus saturated to an energy source which is suitable for producing the polymerization and cross-linking of the monomer.

This treatment is applied to the microporous membrane in such a fashion that the layer of cross-linked polymer is extremely integral and fixed to the walls of the membrane and that it is sufficiently fine that it does not modify any of the physical characteristics of the membrane, in particular the pore diameter and the porosity.

Said treatment is a subject of the Patent Application EP-A561,277 published on the 22nd September 1993 in the name of the company Millipore Corporation and the polymeric microporous membranes resulting from it are marketed by this company under the name of Durapel hydrophobic membranes.

Such membranes have oleophobic and super-hydrophobic properties such as they are not wetted by ethanol or isopropanol, contrary to membranes, even hydrophobic membranes, which are not wetted by water but are wetted by ethanol.

Due to the particularly low surface energy of these membranes, the surface of the liquid phase with which the membrane ought be in contact is in reality extended by an extremely small distance, of the order of a few tenths of a nanometer (or Angström).

Such a spacing avoids all risk of leakage of the liquid; the exchange surface of molecules of the volatile product is practically that of the membrane and as the latter has a pore diameter which is clearly greater than that of these molecules, the volatile product can freely escape through the pores, which play no active role in limiting, controlling or slowing down the diffusion of the volatile product.

The polymeric microporous membranes used according to the present invention are covered over all of their surface, including the interior surface of the pores, with a super-hydrophobic polymer layer, which will allow the surface energy of the membrane to be lowered to a sufficiently low value so that it is not wetted by ordinary alcohol or ethanol.

The surface energy of these membranes is clearly lower than 1.8 x 10⁻² N/m. These membranes will not be wetted by liquid phases containing a volatile product and the surface tension of which can be lower than 2.2 x 10⁻² N/m.

In fact, it is usually accepted that the surface energy of a solid must be less by at least 2 x 10⁻³ and preferably by at least from 3 x 10⁻³ N/m to 5 x 10⁻³ N/m than the surface tension of a liquid, so as not to be wetted by the latter.

The devices according to the invention can have one or two openings covered by said membranes without having the slightest risk of leakage of the liquid, contrary to devices of the prior art in which the membrane was wetted by the liquid.

The surface energy of a membrane can be measured from the polymer used for the membrane, but in a smooth and solid form. The value thus found does not therefore exactly represent the behaviour of this same polymer when it is in a microporous form. Thus, the state of the surface exposed to the liquid in terms of rugosity, pore diameter and porosity will play a role in the wettability of a membrane.

The polymer whose microporous membrane is constituted by a naturally hydrophobic polymer such as a fluorinated polymer such as polyvinylidene difluoride (PVDF), polypropylene (PP), polyethylene (PE), ultra high density polyethylene (UPE), perfluoroalkylene (PFA), polyether sulphone (PES), a polyamide (Nylon®) or polytetrafluoroethylene (PTFE) or other polymers, which are known for allowing water vapour to pass through without allowing water in liquid form.

These polymers are used to produce microporous membranes the pore diameter of which must be comprised between 0.1 and 5 micrometers. In fact, below this lower limit, the membrane risks interfering with the transfer of the volatile product by limiting its passage through pores which are too small. And above 5 micrometers, the membrane is no longer of sufficient strength and becomes too fragile. As a result of this range of pore diameters, the membrane in no way limits the passage of the gas molecules of the volatile product, the diameters of which are 100 to 1,000 times smaller. The membrane behaves as if it is invisible and does not interfere in the evaporation and diffusion processes. In a container one wall of which is replaced by such a membrane, the diffusion of the volatile product takes place in the same fashion as if there was no membrane, by simple evaporation and natural diffusion defined by the saturated vapour pressures of the liquids and the ambient temperature.

In fact, this liquid does not wet the super-hydrophobic membrane and it finds itself a few tenths of a nanometer (a few Angströms) for the surface of the liquid. Under these conditions the membrane is found in the gas phase close to the liquid phase and, due to the size of the pores, all gas exchanges between the molecules which have just vaporized and the outside atmosphere will occur normally without constraint.

Preferably, the polymeric microporous membranes have this range of pore diameters, also having a porosity, i.e. an empty volume or a percentage of empty volume relative to the volume of the membrane, which is comprised between 50 and 90%.

This significant empty volume is necessary to not obstruct or impair the evaporation of the volatile product through the membrane.

The diffusion device for a volatile product according to the present invention is illustrated in the attached figures.

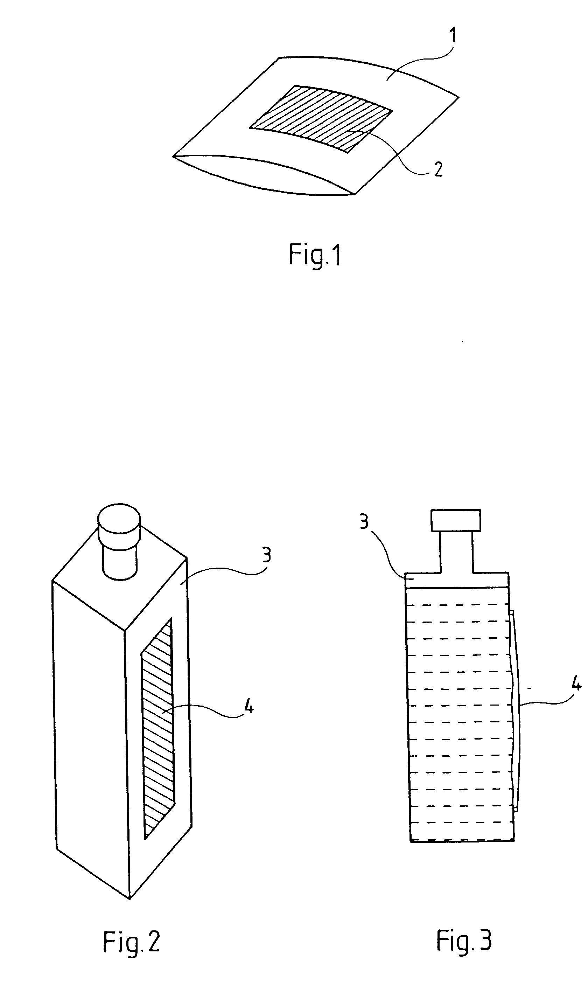

- Figure 1 shows a flexible plastic bag (1) containing a solution of volatile product, such as a perfume, and containing a super-hydrophobic microporous membrane (2) glued or welded (hot, by ultrasound or by high frequency) over an opening cut in the wall of the bag.

Such a bag can be pre-filled and sealed once filled by the liquid phase. The membrane (2) can be protected before use by a non-porous film which can be peeled off (not shown). These bags (1) can hold from 1 to 100 ml and are intended to be carried by an individual or to be placed in a location which one wishes to perfume without risk of wetting.

Figures 2 and 3, show schematically a rigid plastic container (3) which can hold from 10 to 2000 ml, one or more of the vertical walls of which depending on the intensity required of the volatile product, contain openings over which super-hydrophobic microporous membranes (4) are glued or welded.

These containers (3) can be places on an item of furniture or other (Figure 3) or be suspended (Figure 2).

The main advantages of these rigid plastic containers (3) are, on the one hand, to be able to have a large liquid/air interface even for a container with reduced dimensions and, on the other hand, to be able to produce different geometries for this interface.

In fact, when a bottle of rectangular section, full of liquid and open is used, the only liquid/air interface available corresponds to the few cm² of the bottle neck. When the bottle is half-full the interface is larger and corresponds to the cross section of the bottle. On the other hand, if one of the vertical sides of the bottle is replaced by a membrane corresponding to the criteria of the present invention, an interface is immediately obtained which is equal to the surface area of this side, namely 5 to 50 times larger than in the previous example.

Another important advantage is the possibility of finally making available a vertical interface, which does not exist in nature, where all liquid/air interfaces are always horizontal, except for waterfalls which must be considered as moving interfaces.

This type of vertical interface is particularly interesting in the use which is carried out according to the invention, since it allows a much better evaporation flow of the volatile product present in the liquid phase to be obtained.

In fact, while the evaporation and diffusion in such a tight container (3) is the same as for open containers having the same surface of liquid, it is not so for the convection (or put more simply the air currents) which play an essential role in the transportation of the volatile product in the ambient air and therefore in the intensity finally obtained from the same volume of liquid phase.

Therefore, a vertical interface will allow a much better diffusion by convection of the molecules of the volatile product in a room than a horizontal interface of the same surface area.

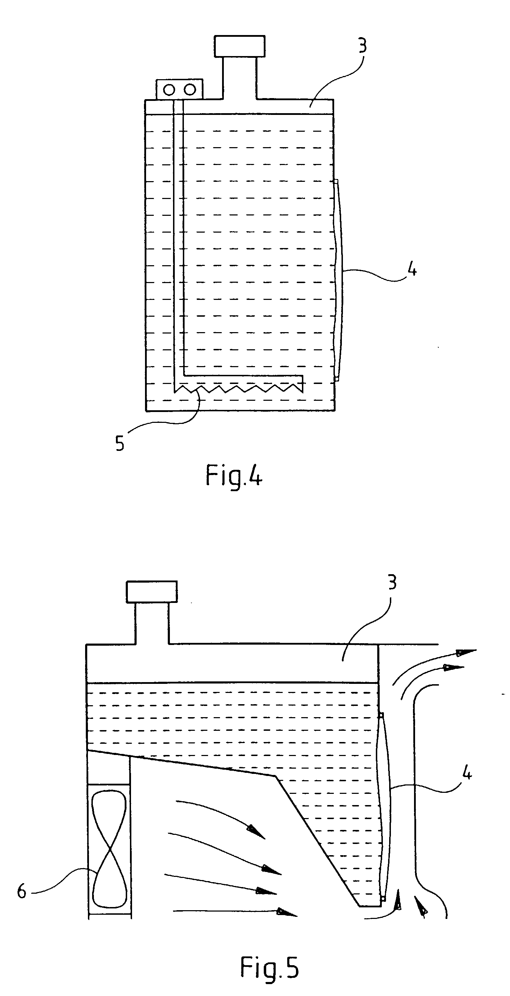

When a yet greater intensity of diffusion is sought, it is possible with rigid plastic containers, either to accelerate the evaporation by arranging a heating means (5), located inside the container and in contact with the liquid phase (see Figure 4), or to accelerate the convection by making available a small fan (6) the air flow of which will sweep the surface of the membrane (see Figure 5), or to make available these two devices at the same time.

Such devices can be used continuously or in an intermittent fashion and in this way a desired intensity of diffusion of volatile product can be obtained on demand.

The diffusion devices according to the invention can be used for the diffusion of perfume, but they are also suitable for the diffusion of deodorant substances which capture odours, insecticides or other repellent products, or disinfectant or bactericidal products, in particular in hospitals or public places.

The membrane (4) can be protected on its external side by a protection element, which can be a screen which allows dosing of the diffusion of the volatile product.

## Claims

1. Device for the diffusion of a volatile product presented in liquid form in a container (3), characterized in that one of the walls of said container, in contact on one side with the liquid phase where the volatile product is found and on the other side with the outside where one wishes to diffuse the volatile product, comprises a polymeric microporous membrane (4) the pore diameter of which is comprised between 0.1 and 5 micrometers and that has been subjected to a treatment to render the membrane super-hydrophobic and oleophobic.

2. Device according to claim 1, characterized in that the microporous membrane (4) has an empty volume or percentage of pores comprised between 50 and 90%.

3. Device according to claim 1 or 2, characterized in that the polymeric microporous membrane (4) has been rendered super-hydrophobic and oleophobic in such a fashion as not to be wetted by ethanol and isopropanol.

4. Device according to any one of the preceding claims characterized in that the microporous membrane (4) is made of polyolefin, polyamide, polystyrene, fluorinated polymer, polysulphone, polyester, polycarbonate, of cellulose or polyvinyl chloride material, having been subjected to a polymerization and cross-linking treatment of a non-saturated monomer having a fluoroalkyl group, in such a fashion as not to be wetted by ethanol and isopropanol.

5. Device according to any one of the preceding claims, characterized in that the container is a flexible plastic bag.

6. Device according to any one of claims 1 to 4, characterized in that the container (3) is a rigid bottle having at least one opening covered with said super-hydrophobic microporous membrane in one vertical wall.

7. Device according to any one of the preceding claims characterized in that the container (3) contains a heating means (5) or a fan (6) to accelerate the evaporation or convection of the volatile product through the membrane.

8. Device according to any one of the preceding claims characterized in that the volatile product is a perfume, a deodorant, an insecticide, a disinfectant product or a bactericidal product.

9. Device according to any one of the preceding claims characterized in that the super-hydrophobic microporous membrane (4) is protected on its external side by a protection element.

10. Device according to claim 9 characterized in that the protection element is a screen which allows dosing of the diffusion of the volatile product.
